# EUROPEAN PATENT SPECIFICATION

(11) **EP 2 440 939 B1**
(45) Date of publication and mention of the grant of the patent: **13.04.2016**
(21) Application number: 10778706.1
(22) Date of filing: 09.06.2010
(51) Int. Cl.: G01N 33/94

(54) **ASPIRIN ASSAY**
ASPIRIN-ASSAY
DOSAGE DE L'ASPIRINE

(30) Priority: 10.06.2009 GB 0909925
(43) Date of publication of application: 18.04.2012
(73) Proprietor: Randox Laboratories Ltd., Crumlin, County Antrim BT29 4QY (GB)
(72) Inventor: BENCHIKH, Elouard, Crumlin Antrim BT29 4QY (GB); FITZGERALD, Stephen Peter, Crumlin Antrim BT29 4QY (GB); INNOCENZI, Paul John, Crumlin Antrim BT29 4QY (GB); LOWRY, Philip Andrew, Crumlin Antrim BT29 4QY (GB); MCCONNELL, Ivan Robert, Crumlin Antrim BT29 4QY (GB)
(86) International application number: PCT/GB2010/050962
(87) International publication number: WO 2010/142984

(56) References cited:
- PATEL D K ET AL: "COMPARATIVE METABOLISM OF HIGH DOSES OF ASPIRIN IN MAN AND RAT" XENOBIOTICA, TAYLOR AND FRANCIS, LONDON, GB, vol. 20, no. 8, 1 January 1990 (1990-01-01), pages 847-854, XP009144207 ISSN: 0049-8254
- PATEL D K ET AL: "METABOLISM OF ASPIRIN AFTER THERAPEUTIC AND TOXIC DOSES" HUMAN AND EXPERIMENTAL TOXICOLOGY, MCMILLAN, BASINGSTOKE, GB, vol. 9, no. 3, 1 January 1990 (1990-01-01), pages 131-136, XP009144206 ISSN: 0960-3271
- RUMBLE R H ET AL: "Metabolism of salicylate during chronic aspirin therapy" BRITISH JOURNAL OF CLINICAL PHARMACOLOGY, BLACKWELL SCIENTIFIC PUBL, GB, vol. 9, no. 1, 1 January 1980 (1980-01-01), pages 41-45, XP009144203 ISSN: 0306-5251
- ZAUGG SANDRA ET AL: "Determination of salicylate, gentisic acid and salicyluric acid in human urine by capillary electrophoresis with laser-induced fluorescence detection" JOURNAL OF CHROMATOGRAPHY B, vol. 752, no. 1, 5 March 2001 (2001-03-05), pages 17-31, XP002621140 ISSN: 0378-4347

## Description

### FIELD OF THE INVENTION

The invention relates to improved methods for the monitoring and quantification of aspirin levels for the purposes of therapeutic drug monitoring and overdose incidents using the ratio of salicylic acid to salicyluric acid in urine.

### BACKGROUND OF THE INVENTION

Aspirin (common scientific name acetylsalicylic acid, IUPAC name 2-acetoxybenzoic acid), is one of the most used drugs, possessing analgesic, anti-pyretic and antiinflammatory properties. In addition to its common use as a treatment for fever and pain, it is clinically used to treat stroke, myocardial infarction, angina, colorectal cancer and rheumatoid arthritis. Its effectiveness as a therapeutic drug is constrained by a narrow concentration range which in serum is a level of approximately 150-300 mg/l. Concentrations below this level are likely to be sub-therapeutic, while concentrations exceeding 300 mg/l are considered to be toxic.

The pharmacokinetics of aspirin have been studied in detail. Ingestion of a therapeutic amount of aspirin is followed by rapid hydrolysis to salicylic acid (SA) with peak levels of salicylic acid occurring after 1-2 hours. Within 2 hours only trace levels of aspirin remain. The half-life of salicylic acid is approximately 6 hours in serum, its hepatic metabolism following several pathways. The principal salicylic acid metabolite is the glycine conjugate salicyluric acid (SUA) which makes up approximately 75% of excreted product. The other main excretory products are gentisic acid (GA - approximately 1%), salicylphenolic glucuronide (SPG - approximately 8%) and salicylacyl glucuronide (SAG - approximately 5%). Unchanged salicylic acid is excreted at a level of approximately 10%. Formation of SUA and SPG follow Michaelis-Menten or zero-order kinetics, while SAG and GA follow first-order kinetics. This means that as the amount of ingested aspirin increases, the SUA and SPG pathways become saturated and the amount excreted levels off leading to an increase in the amount of product excreted as GA, SAG and especially SA.

The availability of aspirin in numerous over-the-counter products and its wide-spread therapeutic use means that aspirin is commonly found in unwell and drug overdose patients presenting at hospital emergency departments. The overdose patients can be categorised as intentional and accidental, the latter often represented by young children and the elderly. There are several methods for monitoring aspirin therapy and determining overdose such as high performance liquid chromatography (HPLC), liquid chromatography-mass spectrometry (LC-MS) or spectrophotometric methods using enzymes and complexation reagents. These methods have drawbacks such as being non-specific, having lengthy assay times, requiring expensive equipment requirements and specialist operators (HPLC/LC-MS), and requiring time-consuming pre-treatment steps. For example, the enzymatic and complexation assays are usually for use with either serum or plasma and due to their sensitivity are usually inadequate for overdose (OD) determination.

An alternative method for testing for aspirin OD is the use of the immunoassay. The benefits of the immunoassay are manifold, especially its simplicity of execution and affordability. Several immunoassays for salicylate detection have been reported. EP 0194352 refers to a salicylate fluorescence polarization immunoassay; US 5089388 describes an enzymatic immunoassay which detects SA, ASA, GA, 2,3-dihydroxybenzoic acid and methyl salicylate. A lateral flow immunoassay which detects both paracetamol and aspirin has been described (Song and Dou 2003). An EMIT immunoassay for salicylic acid has also been described (Fraser 1983). The current immunoassays measure the 'salicylate' level in serum or plasma. Historically, salicylate referred to salicylic acid although current use of the term generally refers to one or more aspirin metabolites. These metabolites include SA, SUA, SPG, SAG and GA. Aspirin overdose can be defined clinically using a semi-quantitative immunoassay format which measures the serum concentration of salicylates at a defined cut-off concentration. The immunoassays available have several drawbacks such as their non-specific nature (the antibody identifies several metabolites which makes quantitation less precise), sample pre-treatment requirements and, most notably, errors associated with calculations to quantify aspirin concentration (unit conversions and dilution correction calculations). It is also proposed that serum salicylate levels frequently do not reflect the severity of the poisoning (O'Malley 2007). Furthermore, serum testing is invasive. A urinary-based assay would be less invasive and simpler, ideally not requiring a pre-assay sample preparation step.

Aspirin overdose patients usually present at a hospital several hours after drug ingestion in a conscious state, so metabolite detection and determination using a 'non-invasive' urine sample would appear to be a more convenient sample type for testing than blood. If the patient presents in an unconscious state a catheter can be implemented. Use of blood as sample type for salicylate testing may have a historical basis. Done's nomogram concept for aiding in the assessment of drug toxicity based on time of ingestion was originally based on plasma salicylate levels. The Done nomogram concept is now recognised as not being of use in cases of sub-acute and chronic salicylate intoxication (Casarett and Doull's Toxicology, 6^{th} Edition). It has been suggested that if plasma salicylate concentration is to be used in such instances, measurements should be taken every three to four hours until the plasma concentration peaks, as the plasma salicylate concentration at presentation can be an unreliable guide to the severity of poisoning (Greene et al. 2005). Of particular concern regarding current blood-based assays is the occurrence of errors in the calculation of salicylate levels from a suspected overdose patient (Hahn et al. 2000; Goldfrank's Toxicologic Emergencies 2002; Eldridge and Holstege 2006; O'Malley 2007). Errors related to dilution correction calculations and to unit conversions for concentration levels can result in misdiagnosis with potentially fatal consequences for an overdose patient.

Patients presenting with aspirin poisoning can be the young, the elderly, self-medicating individuals or individuals on long-term aspirin therapy. Cases of chronic aspirin poisoning in the elderly can partly be a result of the slower and less regular metabolism associated with older individuals and partly due to the higher likelihood of ingestion of the wrong dose or of additional doses of aspirin. Conversely, individuals on aspirin therapy may be under self-medicating and thus unknowingly be prone to the disease or condition for which they assume they have protection. A solution to the problem of aspirin under-dosing or poisoning in individuals self-medicating or on long-term therapy would be for a self-monitoring or doctor's test to ensure that aspirin is at therapeutic and non- toxic levels. Current salicylate tests cannot meet this need as a blood sample is required.

Taking into account the current short-comings of existing tests for aspirin overdose and therapeutic drug monitoring, a new assay which circumvents these problems is desirable.

### SUMMARY OF THE INVENTION

Disclosed herein is a novel method for detecting non-therapeutic, therapeutic and toxic levels of aspirin in an individual which makes use of the urinary SA to SUA ratio. The use of a ratio (no units) for determining non-therapeutic, therapeutic and toxic levels of aspirin avoids the possibility of an error occurring during mathematical calculation through use of the wrong units or when recalculating the true salicylate concentration to account for sample dilution. Furthermore, the use of a urinary sample compared to a blood sample is less invasive and requires no sample preparation prior to analysis, such as hydrolysis of conjugated salicylic acid metabolites to form salicylic acid. Importantly, the SA to SUA ratio method can also be used at home by individuals on aspirin therapy, especially the elderly, to ensure an optimum level of aspirin is maintained and to avoid toxic levels. An increasing ratio of SA to SUA over time is indicative of increasing levels of aspirin that may be exceeding the optimum therapeutic levels and becoming toxic. Likewise, this method is appropriate for use in doctors' surgeries.

The SA to SUA ratio method for increasingly toxic levels of salicylic acid is guided, but not constrained, by the following values (Patel et al. 1990a & 1990b, Hutt et al. 1986, Ho et al. 1985, Rumbleet et al. 1980 and Gibson et al. 1975): SA : SUA between about 1.00 : 7.50 and about 1.00 : 3.00 indicates a therapeutic concentration, SA : SUA between about 1.00 : 3.00 and about 1.00 : 1.00 indicates a moderately toxic concentration, SA : SUA of greater than about 1.00 : 1.00 indicates a toxic concentration.

The invention also describes a method that uses a cut-off value for SUA in urine to signify a toxic concentration of aspirin or OD. This approach can be supported with multiple measurements of SUA over time to confirm SUA zero order kinetics at toxic aspirin levels or OD.

### Definitions

Aspirin - as aspirin is rapidly metabolised to salicylic acid which is the poisonous active component, any mention of aspirin in a toxic context implicitly refers to salicylic acid, unless otherwise qualified.

Toxic concentration - usually associated with an aspirin overdose event (the terms toxic concentration and OD are used interchangeably and refer to one or both of a single toxic ingestion event or to toxic levels induced by chronic ingestion, unless otherwise stated). The patient will usually have one or more concurrent symptoms such as nausea, vomiting and tinnitus. The general literature commonly describes a toxic concentration as aspirin ingestion of > 300 mg/kg.

Moderately Toxic Concentration (MTC) - a concentration that exceeds the upper level of the recognised aspirin therapeutic range but is not an OD event. Chyka et al (2007) describe a MTC level as aspirin ingestion 150 mg/kg to 300 mg/kg or 6.5 g of aspirin equivalent.

A supra therapeutic concentration of aspirin refers to either a MTC or toxic concentration of aspirin.

Highly specific antibodies - antibodies that preferentially bind to a specific molecule. This preferential binding is verified through cross-reactivity (CR) studies which compares the binding of the antibody to the target molecule compared to structurally-related analogues and common drugs. The preferential binding of the antibodies to the target molecules compared to structurally-related analogues should be of such a magnitude so as not to compromise the value of the ratio and thus the integrity of the diagnosis..

### DESCRIPTION OF DRAWINGS

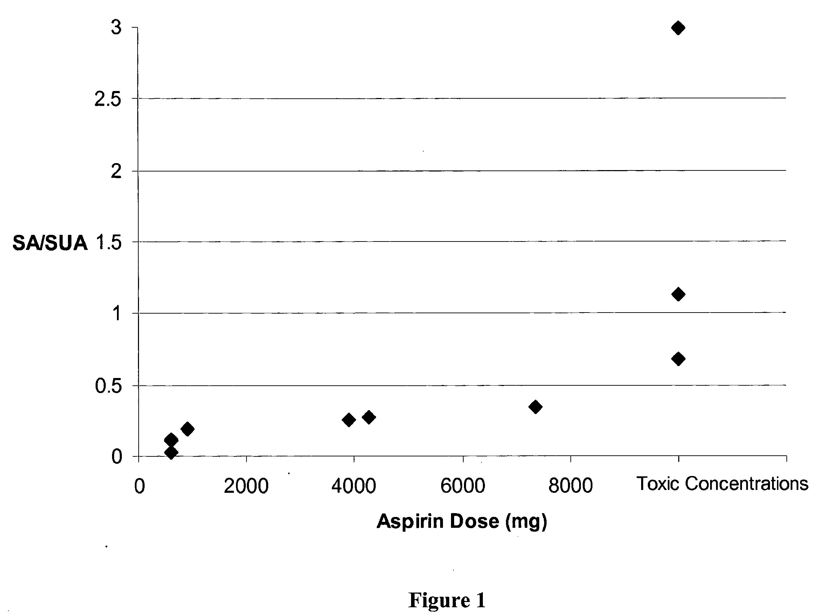

**Figure 1** Urinary salicylic acid (SA) to salicyluric acid (SUA) ratio as a function of increasing aspirin dose.

### DETAILED DESCRIPTION OF THE INVENTION

The invention describes the use of the SA : SUA ratio measured in an *in vitro* urinary sample of an individual to detect sub-therapeutic, therapeutic and toxic levels of aspirin in the individual. The invention also describes novel methods for detecting sub-therapeutic, therapeutic and toxic levels of aspirin in an individual using the urinary SA to SUA ratio. Preferably, it is used for detecting toxic levels of aspirin in an individual, especially overdose levels. The invention is especially useful for application in a testing device for use at home or at the doctor's surgery, to ensure that aspirin levels in individuals on aspirin therapy are at acceptable concentrations, and are not at sub-therapeutic or toxic concentrations. The therapeutic level of salicylic acid in blood of a healthy adult is generally considered to be 150-300 mg/l. This level corresponds to a urinary excretion ratio of SA to SUA of about 1:7.5. As the level of salicylic acid increases either due to chronic aspirin ingestion, or a single large dose, the urinary excretion concentration of SA increases relative to SUA.

As is known to the person skilled in the art, diagnostic assays based on biochemicals are affected by many factors such as the age, diet, sex, medical history, genotype etc., of the presenting patient. Thus, when establishing the ranges and cut-off levels attached to an assay these factors may be taken into consideration. A common approach to determining the optimal performance of a biochemical diagnostic assay is to use the 'sensitivity' and 'specificity' concept. The sensitivity is a measure of how good the test is at correctly identifying true positives, while the specificity is a measure of how good the test is at correctly identifying true negatives. From these values, the concepts of positive predictive value, negative predictive value and ROC curves can be used to assess the performance of the test. These and similar methods can be applied to the current invention.

The SA : SUA ratio method for increasingly toxic levels of aspirin is guided by the following literature values: less than about 1.00 : 3.00 indicates a non-toxic concentration, between about 1.00 : 3.00 and about 1.00 : 1.00 indicates a moderately toxic concentration, and greater than about 1.00 : 1.00 indicates a toxic concentration.

The SA : SUA ratio method for sub-therapeutic and therapeutic levels is guided by the following literature values: less than about 1.00 :7.50 indicates a sub-therapeutic dose and between about 1.00 : 7.50 and about 1.00 : 3.00 indicates a therapeutic concentration. For practical purposes and for diagrammatical representation, these values are represented by the quotient of SA and SUA (Figure 1). These values were derived from studies of patients who were undergoing aspirin therapy or who presented at accident and emergency departments having overdosed on aspirin (see Bibliography).

A first aspect of the invention is the use of the value of the SA : SUA ratio calculated from the levels of salicylic acid and salicyluric acid measured in an *in vitro* urinary sample of an individual as an indicator of sub-therapeutic, therapeutic, moderately toxic and toxic concentrations of aspirin in the individual. Preferably, the SA : SUA ratio is used as an indicator of a *supra*-therapeutic concentration of aspirin in an individual. More preferably, the SA : SUA ratio is used as an indicator of a toxic concentration of aspirin in an individual.

A second aspect of the invention is a method for determining a sub-therapeutic, a therapeutic, a moderately toxic or toxic concentration of aspirin in an individual by measuring the levels of salicylic acid and salicyluric acid in an *in vitro* urinary sample taken from the individual, calculating the ratio of SA : SUA in the sample, and comparing the calculated ratio to one or more ratios of salicylic acid to salicyluric acid indicative of a non-toxic concentration, a moderately toxic or toxic concentration of aspirin. Preferably the ratio of SA : SUA is equal to or less than about 1.00 :7.50 indicates a sub-therapeutic concentration, between about 1.00 : 7.50 and about 1.00 : 3.00 indicates a therapeutic concentration, between about 1.00 : 3.00 and about 1.00 : 1.00 indicates a moderately toxic concentration, and greater than about 1.00 : 1.00 indicates a toxic concentration.

A third aspect of the invention is a method for determining a sub-therapeutic, a therapeutic, a moderately toxic or toxic concentration of aspirin in an individual by measuring the levels of salicylic acid and salicyluric acid in an *in vitro* urinary sample taken from the individual, calculating the ratio of SA : SUA in the sample, and comparing the calculated ratio to one or more ratios of salicylic acid to salicyluric acid indicative of a non-toxic concentration, a moderately toxic or toxic concentration of aspirin. Preferably the ratio of SA : SUA is equal to or less than 1.00 : 7.50 indicates a sub-therapeutic concentration, greater than 1.00 : 7.50 and up to 1.00 : 3.00 indicates a therapeutic concentration, greater than 1.00 : 3.00 and up to 1.00 : 1.00 indicates a moderately toxic concentration, and greater than 1.00 : 1.00 indicates a toxic concentration.

There are several possible analytical formats that could be used to measure the SA : SUA ratio such as HPLC and LC-MS. Preferably the format is an immunoassay as this enables portability and use outside of the clinical laboratory, unlike formats such as LC-MS. Immunoassay formats are varied and could be an ELISA or a lateral flow device, or the SA : SUA ratio test may be part of a multi-analyte array on a biochip or other suitable substrate. A combination of the SA : SUA test with other urine-based aspirin-related tests such as a 11-dehydro thromboxane B2 test for aspirin resistance, either incorporated in a portable device suitable for clinical or self-testing or on a biochip as part of a multi-analyte array for medium to high throughput testing, is an alternative application.

### Bibliography

I.H. Hahn et al. 2000. Academic Emergency Medicine, 7: 1336-1337.
Goldfrank's Toxicologic Emergencies 2002. Editors Goldfrank, Flomenbaum, Lewin, Howland, Hoffman, Nelson. Seventh Edition. Published by McGraw-Hill.
G.F. O'Malley 2007. Emergency Medicine Clinics of North America, 25: 333-346.
S. Wang et al. 2002. An ELISA for the determination of salicylic acid implants using a monoclonal antibody. Plant Science, 162: 529-535.
D.L. Eldridge & C.P. Holstege 2006. Clinics in Laboratory Medicine, 26:13-3.
T. Gibson & G. Zaphiropoulus 1975. Kinetics of salicylate metabolism. Br. J. Clin. Pharmac., 2: 233-238.
R.H. Rumble et al. 1980. Metabolism of salicylate during chronic aspirin therapy. Br. J. Clin. Pharmac., 9: 41-45.
P.C. Ho et al. 1985. The effects of age and sex on the disposition of acetylsalicylic acid and its metabolites. Br. J. Clin. Pharmac., 19: 675-684.
A.J. Hutt et al. 1986. The metabolism of aspirin in man: a population study. Xenobiotica, 16: 239-249.
D.K. Patel et al. 1990a. Comparative metabolism of high doses of aspirin in man and rat. Xenobiotica, 20: 847-854.
D.K. Patel et al. 1990. Metabolism of aspirin after therapeutic and toxic doses. Hum. Exp. Toxicol., 9: 131-136.
W. Song and C. Dou 2003. One-step immunoassay for acetaminophen and salicylate in serum, plasma, and whole blood. J. Anal. Toxicol., 27: 366-371.
A.D. Fraser 1983. Clinical evaluation of the EMIT salicylic acid assay. Ther. Drug Monit., 5: 331-334
P.H. Chyka et al. 2007. Salicylate poisoning: an evidence-based consensus guideline for out of hospital management. Clin. Toxicol. (Phila), 45: 95-131.
S.L. Greene et al. 2005. Postgraduate Medical Journal, 81: 204-216

## Claims

1. A method for determining a sub-therapeutic, a therapeutic, a moderately toxic or toxic concentration of aspirin in an individual by measuring the levels of salicylic acid and salicyluric acid in an *in vitro* urinary sample taken from the individual, calculating the ratio of salicylic acid to salicyluric acid in the sample, and comparing the calculated ratio to one or more ratios of salicylic acid to salicyluric acid indicative of a non-toxic concentration, a moderately toxic or toxic concentration of aspirin in which a ratio of salicylic acid to salicyluric acid of less than about 1.00 :7.50 indicates a sub-therapeutic concentration, between about 1.00 : 7.50 and about 1.00 : 3.00 indicates a therapeutic concentration, between about 1.00 : 3.00 and about 1.00 : 1.00 indicates a moderately toxic concentration, and greater than about 1.00 : 1.00 indicates a toxic concentration.

## Patentansprüche

1. Verfahren zum Bestimmen einer subtherapeutischen, einer therapeutischen, einer mäßig toxischen oder toxischen Konzentration von Aspirin in einem Individuum durch Messen der Gehalte an Salicylsäure und an Salicylharnsäure in einer von dem Individuum entnommenen Urinprobe in vitro, Berechnen des Verhältnisses von Salicylsäure zu Salicylharnsäure in der Probe und Vergleichen des berechneten Verhältnisses mit einem oder mehreren Verhältnissen von Salicylsäure zu Salicylharnsäure, die auf eine nicht toxische Konzentration, eine mäßig toxische oder eine toxische Konzentration von Aspirin hindeuten, wobei ein Verhältnis von Salicylsäure zu Salicylharnsäure von unter etwa 1,00 : 7,50 auf eine subtherapeutische Konzentration hindeutet, zwischen etwa 1,00 : 7,50 und etwa 1,00 : 3,00 auf eine therapeutische Konzentration hindeutet, zwischen etwa 1,00 : 3,00 und etwa 1,00 : 1,00 auf eine mäßig toxische Konzentration hindeutet und über etwa 1,00 : 1,00 auf eine toxische Konzentration hindeutet.

## Revendications

1. Procédé de détermination d'une concentration sous-thérapeutique, thérapeutique, modérément toxique ou toxique d'aspirine chez un individu par mesure des taux d'acide salicylique et d'acide salicylurique dans un échantillon d'urine *in vitro* prélevé sur l'individu, calcul du rapport acide salicylique sur acide salicylurique dans l'échantillon, et comparaison du rapport calculé à un ou plusieurs rapports acide salicylique sur acide salicylurique indicatifs d'une concentration non toxique, d'une concentration modérément toxique ou toxique d'aspirine dans lequel un rapport acide salicylique sur acide salicylurique de moins d'environ 1,00 : 7,50 indique une concentration sous-thérapeutique, entre environ 1,00 : 7,50 et environ 1,00 : 3,00 indique une concentration thérapeutique, entre environ 1,00 : 3,00 et environ 1,00 : 1,00 indique une concentration modérément toxique, et plus d'environ 1,00 : 1,00 indique une concentration toxique.
